# EUROPEAN PATENT APPLICATION

(11) **EP 2 562 242 A1**
(43) Date of publication of application: **27.02.2013**
(21) Application number: 12178107.4
(22) Date of filing: 26.07.2012
(51) Int. Cl.: C12M 1/00

(54) **Fermentation device with intermediate tank**

(30) Priority: 26.08.2011 EP 11179095
(71) Applicant: Thöni Industriebetriebe GmbH, 6410 Telfs (AT)
(72) Inventor: Schennach, Oliver, 6414 Mieming (AT); Köll, Thomas, 6410 Telfs (AT); Krismer, Michael, 6500 Landeck (AT)
(74) Representative: Dilg, Haeusler, Schindelmann Patentanwaltsgesellschaft mbH

(57) **Abstract**

There is provided a fermentation device (100) comprising a fermenter (102) for fermenting organic material and providing a fermenter output material (106). In accordance with an embodiment, the fermentation device (100) comprises an intermediate tank (108) for conditioning the fermenter output material (106), thereby providing a conditioned material (114). The intermediate tank (108) has an inlet (110) for receiving the fermenter output material (106) and an outlet (112) for outputting the conditioned material (114). Further in accordance with an embodiment, the fermentation device (100) comprises a mixing device (116) for mixing the content of the intermediate tank (108). The mixing device (116) may be for example a stirring device located in the intermediate tank (108) or a conveyor for circulating material through the intermediate tank.

## Description

### FIELD OF THE INVENTION

The present invention relates to the field of fermenters for fermenting organic material.

### BACKGROUND OF THE INVENTION

EP 1 072 574 A2 discloses a plug-flow fermenter and a combined conveying/dewatering device directly attached to the fermenter outlet.

EP 1 013 614 A1 discloses a mixing passage for mixing a fermentation product and a flocculation agent. Downstream the mixing passage is an intermediate tank in which the mixture of the fermentation product and the flocculation agent remains until it is discharged into a dewatering device.

In view of the above described situation there exists a need for a fermentation device with improved characteristics.

### SUMMARY OF THE INVENTION

This need may be met by the subject matter according to the independent claims. Advantageous embodiments of the herein disclosed subject matter are described by the dependent claims.

According to an embodiment of a first aspect of the herein disclosed subject matter there is provided a fermentation device comprising a fermenter for fermenting organic material and providing a fermenter output material; an intermediate tank for conditioning the fermenter output material, thereby providing a conditioned material; the intermediate tank having an inlet for receiving the fermenter output material; the intermediate tank having an outlet for outputting the conditioned material; the fermentation device comprising a mixing device for mixing the content of the intermediate tank.

Although usually the content of the fermenter is also subjected to a mixing or stirring operation, it was found that the mixing of the fermenter output material in the intermediate tank may improve the condition of the fermenter output material for subsequent processing, e.g. dewatering. Further, depending on the embodiments realized in the fermentation device, the mixing of the content of the intermediate tank supports further conditioning of the content of the intermediate tank.

A fermenter in accordance with the herein disclosed subject matter may be any fermenter which is operable for fermenting organic material and provides the respective fermenter output material. In particular embodiments of the herein disclosed subject matter are applicable to dry fermentation with a dry substance content up to 50 %. In accordance with a further embodiment, the fermenter is a plug-flow fermenter, wherein the material is moved through the fermenter in an axial direction.

The intermediate tank according to embodiments of the herein disclosed subject matter may have any shape that is suitable for mixing the content of the intermediate tank. According to an embodiment, the intermediate tank has a cylindrical shape.

A mixing device of the intermediate tank in accordance with embodiments of the herein disclosed subject matter is any device that is capable of mixing the content of the intermediate tank. Such mixing devices include for example mechanical mixing devices or hydraulic mixing devices. For example, according to an embodiment the mechanical mixing device is a stirring device having rotatable shaft with mixing paddles mounted thereon. Further examples of a mechanical mixing device for the intermediate tank are mixing paddles mounted to an inner surface of the intermediate tank and a driving unit for rotating the intermediate tank, the thereby allowing mixing of the content of the intermediate tank via the rotation of the intermediate tank.

According to a further embodiment, the mixing device comprises a rotatable shaft and at least one removable paddle being removably affixed to the shaft. A removable paddle has the advantage that the paddle geometry of the mixing device can be adjusted to the content of the fermenter and hence to the content of the intermediate tank. For example, the organic material available for fermentation may vary from season to season and therefore it may be advantageous to adapt the paddle of the mixing device to the respective content of the intermediate tank, depending on the seasonal variations.

A removable paddle has the further advantage that it can easily be changed if the paddle gets broken or otherwise damaged. Due to the relatively small size of the intermediate tank the intermediate tank can be emptied typically in no more than one day, making a change of a paddle for maintenance or adaption purposes economically reasonable.

An example of a hydraulic mixing device is a pump. In accordance with an embodiment, the mixing of the content of the intermediate tank is performed by pumping the content from a mixing outlet of the intermediate tank to a mixing inlet of the intermediate tank, thereby mixing the content of the intermediate tank.

The size of the intermediate tank is relatively small compared to the size of a typical fermenter. According to an embodiment, the intermediate tank is of a size that is adapted to the throughput of the fermenter such that the residence time of the fermenter output material in the intermediate tank is less than one day. According to an embodiment the volume of the intermediate tank is a part of a day-production of the fermenter output material. For example, according to an embodiment, the volume of the intermediate tank is in a range between one half (1/2) of the day-production and a quarter (1/4) of the day-production, e.g. one third (1/3) of the day production.

According to an embodiment, the intermediate tank comprises a conditioning material inlet for introducing conditioning material into the intermediate tank. According to an embodiment, the conditioning material is a structural material. According to a further embodiment, structural material is capable of maintaining its structure during the residence time in the intermediate tank. The structural material may be for example material that comprises pieces of wood, e.g. pieces of branches, twigs, and/or pieces of shrub. The structural material may serve to loosen the content of the intermediate tank. According to an embodiment, the structural material provides passage ways for liquid through the fermenter output material, thereby improving the dewatering of the content of the intermediate tank in an optional subsequent dewatering device.

According to a further embodiment, the fermentation device comprises a transporting system for transporting the conditioning material into the intermediate tank. The transporting system may be of any suitable type. For example, according to an embodiment, the transporting system is a conveyor. It should be understood that the type of the conveyor used may be adapted to the type of conditioning material used. For example, for structural material a screw conveyor may be used for introducing the structural material into the intermediate tank. According to a further embodiment, where pumpable conditioning material, e.g. flocculation material, is used as conditioning material, a pump may be suitable for conveying the conditioning material into the intermediate tank.

According to an embodiment, the outlet for outputting the conditioned material is a first outlet and the intermediate tank further comprises a second outlet configured for discharging sediment from the intermediate tank. According to an embodiment, the second outlet is located at a distance from the first outlet. For example, if the content of the intermediate tank is moved through the intermediate tank in a direction of movement, then according to an embodiment the second outlet for discharging sediment from the intermediate tank is located upstream the first outlet for outputting the conditioned material. Additionally or alternatively, the second outlet is located at a distance from the first outlet in a circumferential direction, e.g. in a direction perpendicular to the direction of movement.

According to a further embodiment, the intermediate tank may include a recess with the second outlet being located in the recess. Such a recess allows to collect sediment in the recess while the remaining content of the intermediate tank is still mixed by the mixing device. In accordance with the herein disclosed subject matter, the sediment includes inter alia material with a density that is higher than the average density of the material in the intermediate tank. For example, in an embodiment, the sediment comprises large pieces of material, such as stones, etc. However, the sediment may also include sand etc.

According to a further embodiment, the fermentation device comprises a conveyor for receiving the sediment from the intermediate tank (hereinafter referred to as sediment conveyor) and conveying the sediment. According to an embodiment, the sediment conveyor is operated intermittently in order to not remove too much conditioned material through the second outlet. For example, if the intermediate tank comprises a recess in its inner surface, the sediment conveyor may be operated if the recess contains a suitable amount of sediment. For example, in an embodiment a sensor for detecting an amount of sediment in the recess may be provided. According to an embodiment, the sensor provides a sensor signal indicative of the amount of sediment in the recess. The sensor signal may be received by a control unit. According to an embodiment, the control unit activates the sediment conveyor if the amount of the sediment contained in the recess is above a certain limit and/or if a size of pieces of the sediment material, e.g. the size of a stone, is above a certain limit.

According to an embodiment, the second outlet is axially located between the inlet and the first outlet.

According to a further embodiment, the fermentation device further comprises a dewatering device for receiving the conditioned material, the dewatering device being operable for dewatering the conditioned material. Due to the conditioning in accordance with embodiments of the herein disclosed subject matter, dewatering of the conditioned material by the dewatering device may be supported and/or improved. According to an embodiment, the dewatering device is a screw press. However, the dewatering device may be of any type suitable for dewatering the conditioned material.

According to an embodiment of a second aspect of the herein disclosed subject matter, a method of operating a fermentation device is provided, the method comprising introducing a fermenter output material into an intermediate tank; and mixing the fermenter output material in the intermediate tank, thereby providing a conditioned material.

According to a further embodiment, the method further comprises introducing conditioning material into the intermediate tank.

According to a further embodiment, die conditioning material is capable of maintaining its structure during the residence time in the intermediate tank.

According to a further embodiment, the method further comprises collecting sediment in the intermediate tank; intermittently removing the collected sediment from the intermediate tank.

According to a further embodiment, the method further comprises dewatering the conditioned material.

According to further embodiments of the second aspect, the method is adapted for providing the functionality of one or more of the aforementioned embodiments and/or for providing the functionality as required by one or more of the aforementioned embodiments, in particular of the embodiments of the first aspect.

According to an embodiment of a third aspect, a control unit for a fermentation device is provided, the control unit being configured for carrying out the method according to an embodiment of the second aspect.

According to an embodiment of a forth aspect, a computer program is provided, the computer program being adapted for, when being executed by a data processor device, controlling the method as set forth in anyone of the embodiments of the second aspect.

As used herein, reference to a computer program is intended to be equivalent to reference to a program element and/or to a computer readable medium containing instructions for controlling a computer system to effect and/or coordinate the performance of the above described method.

The computer program may be implemented as computer readable instruction code by use of any suitable programming language, such as, for example, JAVA, C++, and may be stored on a computer-readable medium (removable disk, volatile or non-volatile memory, embedded memory/processor, etc.). The instruction code is operable to program a computer or any other programmable device to carry out the intended functions. The computer program may be available from a network, such as the World Wide Web, from which it may be downloaded.

The invention may be realized by means of a computer pro-gram respectively software. However, the invention may also be realized by means of one or more specific electronic circuits respectively hardware. Furthermore, the invention may also be realized in a hybrid form, i.e. in a combination of software modules and hardware modules.

In the above there has been described in the following there will be described exemplary embodiment of the subject matter disclosed herein with reference to a fermentation device, a method of operating a fermentation device, a control unit and a computer program. It has to be pointed out that of course any combination of features relating to different aspects of the herein disclosed subject matter is also possible. In particular, some embodiments have been or will be described with reference to apparatus type embodiments, whereas other embodiments have been or will be described with reference to method type embodiments. However, a person skilled in the art will gather from the above and from the following description that, unless otherwise notified, in addition to any combination of features belonging to one aspect also any combination between features relating to different aspects or embodiments, for example even between features of the apparatus type embodiments and features of the method type embodiments is considered to be disclosed with this application.

The aspects and embodiments defined above and further aspects and embodiments of the herein disclosed subject matter are apparent from the examples to be described herein after and are explained with reference to the drawings but to which the invention is not limited.

### BRIEF DESCRIPTION OF THE DRAWINGS

Fig. 1 shows a fermentation device in accordance with embodiments of the herein disclosed subject matter.
Fig. 2 shows a view from above cross-section of a horizontal intermediate tank in accordance with embodiments of the herein disclosed subject matter.
Fig. 3 shows a cross-sectional view of the intermediate tank of Fig. 2 along line III-III.
Fig. 4 shows a fermentation device in accordance with embodiments of the herein disclosed subject matter.
Fig. 5 shows part of a fermentation device in accordance with embodiments of the herein disclosed subject matter.

### DETAILED DESCRIPTION

The illustration in the drawings is schematic. It is noted that in different figures, similar or identical elements are provided with the same reference signs or with reference signs which are different from the corresponding reference signs only differ in the first digit.

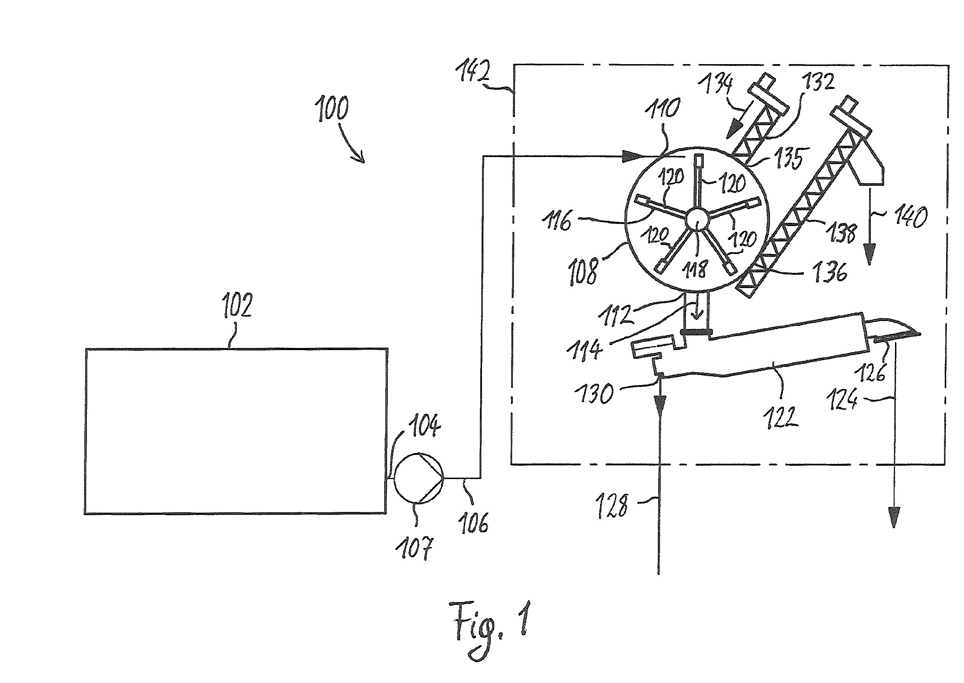

Fig. 1 shows a fermentation device 100 in accordance with embodiments of the herein disclosed subject matter.

The fermentation device 100 comprises a fermenter 102 for fermenting organic material (not shown in Fig. 1). The fermenter 102 has an outlet 104 for providing a fermenter output material indicated at 106 in Fig. 1. The fermentation device 100 further comprises an intermediate tank 108 for conditioning the fermenter output material 106. According to a further embodiment, a conveyor 107, e.g. a discharge pump, is provided for conveying the fermenter output material 106 to the intermediate tank 108. The intermediate tank has an inlet 110 for receiving the fermenter output material 106. Further, the intermediate tank has an outlet 112 for outputting the conditioned material 114. In accordance with an embodiment, the fermentation device 100 comprises a mixing device 116 for mixing the content of the intermediate tank 108. In accordance with an embodiment, the mixing device 116 is a stirring device having a rotatable shaft 118 and pluralities of paddles 120 mounted thereto. The rotatable shaft 118 is rotatable about an access of rotation which extends perpendicular to the drawing plane of Fig. 1. According to an embodiment, the paddles 120 are fixedly mounted to the rotatable shaft 118. According to other embodiments not shown in Fig. 1, the paddles 120 are removably mounted to the rotatable shaft 118. The stirring device 116 may be configured similar to stirring devices for fermenters, except that the dimensions of the stirring device of the intermediate tank 108 are smaller than the typical dimensions of for example a plug-flow fermenter. According to a further embodiment, the fermentation device 100 comprises a dewatering device 122, the dewatering device being adapted for receiving the conditioned material 114, dewatering the conditioned material 114 and providing a dewatered material 124 at its output 126. The remaining liquid 128 is provided at liquid output 130 of the dewatering device 122. It is noted that usually the remaining liquid 128 is referred to as press water. However, the press water still contains organic material and need not be obtained by pressing the conditioned material. Nontheless, in accordance with an embodiment, the dewatering device 122 is a screw press.

In accordance with a further embodiment, the fermentation device 100 comprises a transporting system 132, e.g. in the form of a conveyor, for conveying and introducing conditioning material, indicated at 134, through an inlet 135 into the intermediate tank 108. In accordance with an embodiment, the intermediate tank 108 comprises a further outlet 136 for discharging sediment from the intermediate tank 108. Further in accordance with an embodiment, a conveyor 138 is provided at the further outlet 136 for receiving the sediment from the intermediate tank 108 and conveying the sediment, which is indicated at 140 in Fig. 1. The conveyor 138 for the sediment may be a screw conveyor as shown in Fig. 1. However, other types of conveyors are also possible.

The intermediate tank 108, the conveyors 132, 138 and the dewatering device 122 form a dewatering assembly 142 according to an embodiment of the herein disclosed subject matter.

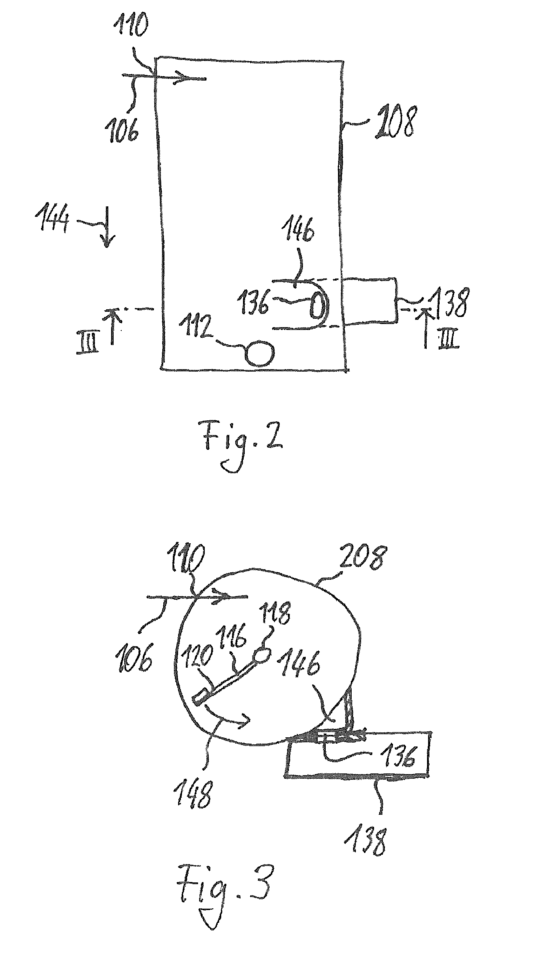

Fig. 2 shows a view from above cross-section of a horizontal intermediate tank 108 in accordance with embodiments of the herein disclosed subject matter.

Generally, the configuration of the intermediate tank 108 in Fig. 2 is similar to the intermediate tank 108 in Fig. 1. In particular, the intermediate tank 208 in Fig. 2 comprises an inlet 110 for receiving the fermenter output material 106 and an outlet 112 for outputting the conditioned material (not shown in Fig. 2). An axial direction is indicated at 144 in Fig. 2. The axial direction 144 indicates the direction of movement of the contents of the intermediate tank 208 during operation. When a portion of the fermenter output material 106 is introduced at the inlet 110, a respective portion of conditioned material is urged through the outlet 112 out of the intermediate tank 208. In Fig. 2 a mixing device is omitted for the sake of clarity. The intermediate tank 208 further comprises a recess 146 which is located at a side portion of the intermediate tank 208. If a stirring device is used for mixing the content of the intermediate tank, such a stirring device would be configured to rotate counterclockwise, thereby moving sediment and heavy particles towards and into the recess 146. Intermittently, a conveyor 138 is operated for discharging the collected sediment. It should be understood that the location of the recess 146 depends on the direction of rotation of the stirring device if a stirring device is used, on the rotational speed of the stirring device, etc. In other embodiments, where a pump is used for mixing the content of the intermediate tank, the recess may be provided for example in a center portion of the device, unless the circulation of material through the intermediate tank provides some driving force which drives the sediment to one side of the intermediate tank (similar to the case for a rotating stirring device 116). Although in Fig. 2 the recess 146 is shown at the right side of the intermediate tank when viewed in a direction opposite to the direction of movement 144, it should be understood that this configuration is only exemplary and that according to other embodiments, the recess 146 may be provided at a different location in the intermediate tank 208, depending on the type of mixing device, its operation parameters and/or depending on the content of the intermediate tank 208.

Fig. 3 shows a cross-sectional view of the intermediate tank 208 of Fig. 2 along line III-III.

In accordance with an embodiment, the recess 146 is located in the right half of the intermediate tank 208. This is due to the fact that the stirring device 116 having a plurality of paddles 120 mounted to a rotatable shaft 118 is rotated counterclockwise as indicated by the arrow 148. This rotational movement of the stirring device 116 shifts the sediment towards the right half of the intermediate tank 208. For this reason it is suitable to arrange the recess 146 in the right half of the intermediate tank. However, a recess in a center portion of the bottom of the fermenter 208 may also be a suitable choice. However, the likelihood of collecting most of the sediment in the intermediate tank is higher if the recess 146 is located at a circumferential position where most of the sediment is found. However, this position may depend e.g. on the viscosity of the content of the intermediate tank, the rotational speed and rotational direction of the stirring device, etc. Therefore, since the recess 146 is provided at a fixed location for a particular intermediate tank, the discharge of the sediment can be optimized by adapting the operating parameters of the stirring device (e.g. rotational speed of the stirring device) to the properties of the content of the intermediate tank. Such a property which might be relevant is for example the viscosity, as noted above.

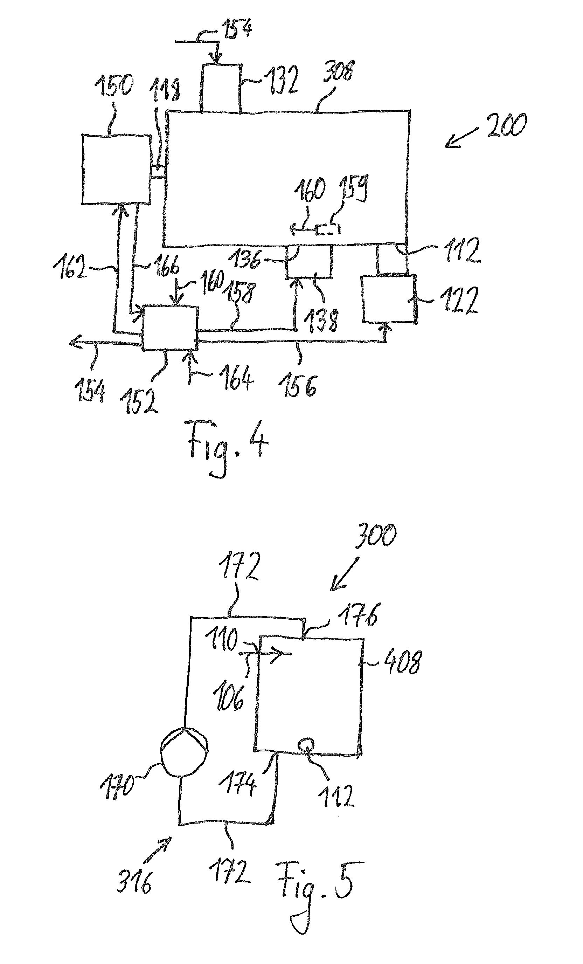

Fig. 4 shows a fermentation device 200 in accordance with embodiments of the herein disclosed subject matter.

In accordance with an embodiment, the fermentation device 200 comprises an intermediate tank 308, configured in accordance with anyone of the embodiments disclosed herein. The fermentation device 200 comprises a mixing device not shown in Fig. 4. In accordance with an embodiment, the mixing device is a stirring device driven by a driving unit 150, e.g. a motor, which is coupled to the rotatable shaft 118 of the stirring device thereby being capable of driving the stirring device. According to an embodiment, the stirring device is located within the intermediate tank 308. However, embodiments of the herein disclosed subject matter, in particular embodiments described below are also combinable with different embodiments of the mixing device, if technically feasible.

In accordance with an embodiment, the fermentation device 200 comprises a sediment conveyor 138 for receiving sediment from the intermediate tank 308 and conveying the sediment. Further, the fermentation device 200 comprises a dewatering device 122. The fermentation device 200 further comprises an input conveyor 132 for conveying conditioning material into the intermediate tank 308.

In accordance with an embodiment, the fermentation device 200 comprises a control unit 152 for controlling the fermentation device 200.

According to an embodiment, the control unit 152 is adapted for outputting a first control signal 154, the first control signal 154 being adapted for effecting introduction of conditioning material into the intermediate tank 308. For example, according to an embodiment the first control signal 154 activates the input conveyor 132 for conveying conditioning material into the intermediate tank 308. According to a further embodiment, the control unit 152 is configured for outputting a second control signal 156, the second control signal 156 being effecting dewatering of a conditioning material provided at an outlet 112 of the intermediate tank. For example, according to an embodiment the second control signal 156 is adapted for activating the dewatering device 122, the dewatering device 122 in response hereto pressing the conditioned material received from the outlet 112 in order to obtain dewatered material and press water, e.g. as described with regard to Fig. 1.

According to a further embodiment, the control unit 152 is adapted for outputting a third control signal 158, the third control signal 158 being adapted to effect discharge of a sediment of the intermediate tank 308. For example, according to an embodiment the third control signal 158 is configured for activating the sediment conveyor 138 which in response hereto conveys sediments received from the sediment output 136 away from the intermediate tank 308.

According to an embodiment, the control unit 152 is configured for outputting the third control signal 158 intermittently. For example, according to an embodiment the control unit 152 is configured for outputting the third control signal at predetermined time intervals. According to other embodiments, a point in time where the third control signal 158 is outputted to the sediment conveyor 138 is determined by the control unit 152 depending on operating conditions of the fermentation device 200. For example, according to an embodiment the control unit 152 determines the point in time for outputting the third control signal depending on the amount or the type of sediment contained in a recess of the intermediate tank 308. To this end, a respective sediment sensor 159, for example an ultrasonic sensor, is provided, the sediment sensor 159 being adapted for providing an sensor signal 160 to the control unit 152, the sensor signal 160 being indicative of at least one sediment parameter, e.g. the amount and/or the type of sediment in the recess.

According to an embodiment, the sediment sensor is located at the recess, e.g. at an outer surface of a wall portion forming the recess.

According to a further embodiment, where no recess is provided in the intermediate tank, the sediment sensor 159 may be attached to a wall of the intermediate tank at a location where sediment is supposed to be found. According to an embodiment, in such a case, the sediment sensor may be provided in the vicinity of the sediment outlet 136 (which according to an embodiment is also present without a recess 146).

According to a further embodiment, the control unit 152 is configured for outputting a forth control signal 162 which is adapted to control the rotational speed of the motor 150 and hence of the rotatable shaft 118 of the stirring device. As mentioned above, the rotational speed as well as the rotational direction of the stirring device determines the location where most of the sediment is found. According to an embodiment, the rotational speed and the rotational direction of the motor 150 is controlled by the control signal 162 so as to move the sediment to a circumferential position where the recess of the intermediate tank is located. In other embodiments, the rotational speed and the rotational direction of the motor 150 may be controlled by the forth control signal 162 in order to move the sediment to a position where a sediment sensor is located.

According to an embodiment, the rotational speed of the motor 150 is controlled by the control unit 152 according to a user input 164. Such a user input may for example indicate the type of the fermenter output material, the dry substance content of the fermenter output material, etc. In such a case, the control unit may be adapted for determining in response to such a user input 152 operating parameters and the control signals 154, 156, 158, e.g. by use of a look-up table or by use of a predefined functional relationship between the user input and the control signals 154, 156, 158, 162. According to a further embodiment, the motor 150 is configured for providing a sensor signal being indicative of the driving torque provided by the motor 150 to the rotatable shaft 118 of the stirring device, the driving torque being indicative of the viscosity of the content of the intermediate tank 308. Such a sensor signal (torque indicating signal) is indicated at 166 in Fig. 4. According to other embodiments, a torque indicating signal or a viscosity indicting signal, indicative of a viscosity of the material in the intermediate tank, may be provided by any other suitable sensor. According to an embodiment, the control unit 152 is configured for determining the control signals 154, 156, 158, 162 depending on sensor signals 160, 166 and/or user input 164.

In accordance with an embodiment, the control unit 152 comprises a data processor device including at least one processor, the processor device being capable of carrying out a computer program being adapted for providing the desired functionality of the control unit as described above.

According to a further embodiment, the control unit 152 also provides a control signal for a conveyor 107 being provided for conveying the fermenter output material 106 to the intermediate tank 108, as described with regard to Fig. 1.

Fig. 5 shows part of a fermentation device 300 in accordance with embodiments of the herein disclosed subject matter.

Fig. 5 shows an intermediate tank 408 having an inlet 110 for receiving the fermenter output material 106 and an outlet 112 for outputting the conditioned material. The fermentation device 300 further comprises a mixing device 316, the mixing device 316 comprising a conveyor 170, e.g. a pump, and a circulation path 172 for suction of material through a circulation outlet 174 out of the intermediate tank and pushing the material through a circulation inlet 176 back into the intermediate tank 408. By this circulation of material contained in the intermediate tank 408, a mixing of the content of the intermediate tank 408 is achieved. In accordance with further embodiments, any element described with regard to other embodiments of fermentation devices disclosed herein can also be used in conjunction with the mixing device 316 described with regard to Fig. 5. Moreover, even other mixing devices may be used as long as they provide for mixing of the content of the intermediate tank. It is noted that in some embodiments the respective features of the fermentation device have to be adapted to the type of mixing device used. For example, the recess 146 described with regard to Fig. 2 and Fig. 3 may be provided in a center portion of the bottom of the intermediate tank if the mixing device only produces an axial movement through the intermediate tank, but no force transverse to the flow of material in the axial direction. However, such adaption can easily be performed for the type of mixing device used without departing from the principles of the embodiments of the herein disclosed subject matter.

It should be noted that any entity disclosed herein, e.g. components, units and devices, are not limited to a dedicated entity as described in some embodiments. Rather, the herein disclosed subject matter may be implemented in various ways and with various granularities on device level or software module level while still providing the desired functionality. Further, it should be noted that according to embodiments a separate entity (e.g. a software module, a hardware module or a hybrid module (combined software/hardware module)) may be provided for each of the functions of the control unit disclosed herein. According to other embodiments, an entity (e.g. a software module, a hardware module or a hybrid module) is configured for providing two or more functions as disclosed herein. As a further example, also device component may be provided at various granularity levels. For example, a conveyor disclosed herein may in fact be realized by two or more conveyor devices. Likewise, a pump disclosed herein may be realized by two or more pumping devices or by a single pumping device.

It should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

In order to recapitulate the above described embodiments of the present invention one can state:
There is provided a fermentation device comprising a fermenter for fermenting organic material and providing a fermenter output material. In accordance with an embodiment, the fermentation device comprises an intermediate tank for conditioning the fermenter output material, thereby providing a conditioned material. The intermediate tank has an inlet for receiving the fermenter output material and an outlet for outputting the conditioned material. Further in accordance with an embodiment, the fermentation device comprises a mixing device for mixing the content of the intermediate tank. The mixing device may be for example a stirring device located in the intermediate tank or a conveyor for circulating material through the intermediate tank.

### List of reference signs

- 100: fermentation device
- 102: fermenter
- 104: fermenter outlet
- 106: fermenter output material
- 107: conveyor
- 108: intermediate tank
- 110: inlet
- 112: outlet
- 114: conditioned material
- 116: mixing device
- 118: rotatable shaft
- 120: paddle
- 122: dewatering device
- 124: dewatered material
- 126: output
- 128: remaining liquid
- 130: liquid outlet
- 132: transporting system (input conveyor) for conditioning material
- 134: conditioning material
- 135: inlet for conditioning material
- 136: further outlet for sediment
- 138: sediment conveyor
- 140: sediment
- 142: dewatering assembly
- 144: axial direction
- 146: recess
- 148: counterclockwise rotation
- 150: driving unit
- 152: control unit
- 154: first control signal
- 156: second control signal
- 158: third control signal
- 159: sediment sensor
- 160: sensor signal
- 162: fourth control signal
- 164: user input
- 166: torque indicating signal
- 170: conveyor for circulating material through the intermediate tank
- 172: circulation path
- 174: circulation outlet
- 176: circulation inlet
- 200: fermentation device
- 208: intermediate tank
- 300: fermentation device
- 308: intermediate tank
- 316: mixing device
- 408: intermediate tank

## Claims

1. Fermentation device (100, 200, 300) comprising:
- a fermenter (102) for fermenting organic material and providing a fermenter output material (106); and
- an intermediate tank (108, 208, 308, 408) for conditioning the fermenter output material (106), thereby providing a conditioned material (114);
- the intermediate tank (108, 208, 308, 408) having an inlet (110) for receiving the fermenter output material (106);
- the intermediate tank (108, 208, 308, 408) having an outlet (112) for outputting the conditioned material (114);
- the fermentation device (100, 200, 300) comprising a mixing device (116, 316) for mixing the content of the intermediate tank (108, 208, 308, 408); and
- a dewatering device (122) for receiving the conditioned material (114), the dewatering device (122) being operable for dewatering the conditioned material (114).

2. Fermentation device according to claim 1, further comprising a conditioning material inlet (135) for introducing conditioning material (134) into the intermediate tank (108, 208, 308, 408).

3. Fermentation device according to claim 2, further comprising a transporting system (132) for transporting the conditioning material (134) into the intermediate tank (108, 208, 308, 408).

4. Fermentation device according to any one of claims 2 or 3, the conditioning material (134) being a structural material and/or flocculation material.

5. Fermentation device according to any one of the preceding claims,
- the outlet (112) being a first outlet; and
- the intermediate tank (108, 208, 308, 408) having a second outlet (136) configured for discharging sediment (140) from the intermediate tank (108, 208, 308, 408).

6. Fermentation device according to claim 5, further comprising a conveyor (138) for receiving the sediment (140) of the intermediate tank (108, 208, 308, 408) and conveying the sediment (140).

7. Fermentation device according to claim 5 or 6, wherein second outlet (136) is axially located between the inlet (110) and the first outlet (112).

8. Fermentation device according to any one of the preceding claims, the mixing device (116) comprising
- a rotatable shaft (118); and
- at least one removeable paddel (120) being removably affixed to the shaft (118).

9. Method of operating a fermentation device, the method comprising:
- introducing a fermenter output material (106) into an intermediate tank (108, 208, 308, 408);
- mixing the fermenter output material (106) in the intermediate tank (108, 208, 308, 408), thereby providing a conditioned material (114);
- dewatering the conditioned material (114).

10. Method according to claim 9, further comprising introducing conditioning material (134) into the intermediate tank (108, 208, 308, 408).

11. Method according to any one of claims 9 to 10, further comprising
- collecting sediment (140) in the intermediate tank (108, 208, 308, 408);
- intermittently removing the collected sediment (140) from the intermediate tank (108, 208, 308, 408).

12. Control unit (152) for a fermentation device, the control unit being configured for carrying out the method according to any one of claims 9 to 11.

13. Computer program being adapted for, when being executed by a data processor device, controlling the method as set forth in any one of the claims 9 to 11.
